# EUROPEAN PATENT SPECIFICATION

(11) **EP 3 389 484 B1**
(45) Date of publication and mention of the grant of the patent: **31.03.2021**
(21) Application number: 15910878.6
(22) Date of filing: 14.12.2015
(51) Int. Cl.: A61B 5/021, A61B 5/0215, A61B 5/02, G01C 15/00

(54) **TRANSDUCER CLIP**
WANDLER-CLIP
ATTACHE POUR TRANSDUCTEURS

(43) Date of publication of application: 24.10.2018
(73) Proprietor: Edwards Lifesciences Corporation, Irvine, CA 92614 (US)
(72) Inventor: CARROLL, Sean, Irvine CA 92614 (US); WINE, Jason, A., Irvine CA 92614 (US)
(74) Representative: Eisenführ Speiser
(86) International application number: PCT/US2015/065621
(87) International publication number: WO 2017/105397

(56) References cited:
- WO-A1-02/074398
- WO-A1-2005/094952
- US-A- 5 280 789
- US-A- 5 339 810
- US-A- 5 388 619
- US-A- 5 758 657
- US-A- 6 071 243
- US-A1- 2007 213 623
- US-A1- 2007 282 272
- US-A1- 2009 294 604
- US-A1- 2010 222 793
- US-A1- 2014 007 408
- US-S- D 678 533

## Description

### BACKGROUND

Arterial blood pressure transducers are used to monitor patients in hospitals, nursing facilities, and other medical treatment facilities. These transducers are used in a system that generally includes an IV bag, a first section of tubing that extends from the bag to the transducer, a second section of tubing that extends from the transducer to the patient, and a cable that extends from the transducer to a device that receives signals from the transducer to monitor the patient. Often patients are monitored using multiple, different transducers. A patient may be monitored with a first transducer that monitors a central venous pressure (CVP), a second transducer that monitors a pulmonary artery (PA), and a third transducer that monitors a left arterial pressure (LAP). Organizing the transducers and the various tubing sections and cables is often cumbersome for the patient and for the medical staff. Further, one or both of the patient and medical staff are required to move and rearrange the various transducer systems when the patient moves locations (e.g., moves from a first examination room to a second examination room), or adjusts their physical position, such as while they are lying in bed or upon getting out of bed.

While this is one example, there are other various medical devices that are employed to monitor patients that require placement relative to the placement with associated space management issues. Accordingly, there is a need for an improved device for holding and organizing medical devices, such as transducers, used for monitoring patients.

A pressure transducer positioning system is known from US 5,769,083.

### SUMMARY

The present application is directed to a clip for receiving one or more medical devices, such as transducers used to measure arterial blood pressure. The clip is also configured to be attached to different types of objects, and may be attached at various orientations that accommodate the position and/or activity of the patient. In some embodiments, the clip may be mounted on poles or other objects that are not vertical, but the clip may still maintain the vertical alignment of transducers on the clip with a patient (e.g., with the right atrium of the patient's heart). The clip provides simplicity and convenience to the user by providing ease of adjustment, relieving space on the clip for other components otherwise used by an alignment device, the ability to quickly attach to multiple pole sizes and/or orientations and may allow single-hand adjustment of the clip.

A clip in accordance with the invention is defined in claim 1.

Preferred embodiments are defined in the dependent claims.

The features, functions, and advantages that have been discussed may be achieved independently in various embodiments of the present invention or may be combined with yet other embodiments, further details of which can be seen with reference to the following description and drawings.

### BRIEF DESCRIPTION OF THE DRAWINGS

Having thus described embodiments of the system in general terms, reference will now be made to the accompanying drawings, where:
Figure 1 depicts a conventional method of measuring blood pressure;
Figure 2 depicts a catheter being inserted into a patient's heart to facilitate measuring blood pressure;
Figure 3 depicts a method of measuring blood pressure in accordance with an aspect of the present invention;
Figure 4A depicts a perspective view of an exemplary clip in accordance with an aspect of the present invention;
Figure 4B depicts a side view of an exemplary clip in accordance with an aspect of the present invention;
Figure 4C depicts a rear view of an exemplary clip in accordance with an aspect of the present invention;
Figure 5 depicts an exemplary alignment device in accordance with an aspect of the present invention;
Figure 6 depicts another exemplary alignment device in accordance with an aspect of the present invention;
Figure 7 depicts an exemplary rotatable alignment device in accordance with an aspect of the present invention;
Figure 8 depicts another exemplary rotatable alignment device in accordance with an aspect of the present invention;
Figure 9A depicts a top view of another exemplary clip in accordance with an aspect of the present invention;
Figure 9B depicts a top view of yet another exemplary clip in accordance with an aspect of the present invention;
Figure 10A depicts a side view of an exemplary attachment location formed from a rotatable structure in accordance with an aspect of the present invention;
Figure 10B depicts a top view of an exemplary attachment location formed from a rotatable structure in accordance with an aspect of the present invention;
Figure 10C depicts a top view of another exemplary attachment location formed from a rotatable structure in accordance with an aspect of the present invention;
Figure 11A depicts a top view of yet another exemplary clip in accordance with an aspect of the present invention;
Figure 11B depicts a cross-sectional side view of yet another exemplary clip in accordance with an aspect of the present invention; and
Figures 12A-12C depict a top view of an exemplary clip in accordance with another aspect of the present invention.

### DETAILED DESCRIPTION

The device as described in the present disclosure is particularly useful in a patient monitoring environment, whereby the device may be used to retain and position one or more medical devices relative to a patient. It is of particular use where accurate alignment of a medical device relative to a patient is required. For example, alignment may be required to ensure the sensors of the monitoring device are aligned with a sensing target on a patient. Alignment may also be required to position a medical device above a patient for proper gravity flow of fluids or below a patient for fluid removal. While various examples of use of the device are contemplated, to provide a full disclosure, the device is described herein within the context of use with retaining an arterial blood pressure transducer at a position relative to the patient for accurate heart function monitoring.

In one aspect, the present application is directed to a medical clip for receiving one or more transducers (e.g., transducer modules). The clip and transducer are configured to attach to a variety of different objects, such as but not limited to a pole, a bed, curtains, drapes, and clothing. The clip generally comprises first and second arms that are pivotally connected together. The clip is configured to be movable between an open orientation to extend around an object, and a closed orientation to attach to the object. One or more connectors are positioned along the outer sides of the arms to receive and/or connect and/or attach with a medical device, such as a transducer. The connectors are configured to provide a secure and releasable connection with the medical device. In one aspect, the arms or outer side of an arm of the clip can be configured to rotate while the clip is attached to the object. In another aspect, the clip includes an alignment device (e.g., a laser or other light emitting device that can be used for aligning the clip relative to a patient). The alignment device is coupled to one of the first or second arms or the outer or inner side of an arm of the clip. In another aspect, the clip includes a housing coupled to an arm, the housing including an alignment device that is configured to rotate within the housing while the clip is attached to an object. The alignment device can include a self-leveling feature. The present application is also directed to methods of using the clip and the alignment device.

To obtain the most accurate measurement of CVP, the zero point of a manometer or a given measuring point on a transducer (or its module) should be closely vertically aligned with a desired point on the patient, such as a location corresponding to the patient's right atrium and/or vena cava. If the vertical level of a point at which pressure is measured is significantly below the right atrium, the CVP reading may be too high. On the other hand, if the vertical level of this measuring point is significantly higher than the right atrium, the CVP reading may be too low.

In addition, if the vertical level of one of the right atrium and the measuring point significantly changes over time while the other of these vertical levels remains the same, CVP may appear to trend upwardly or downwardly when actual CVP has remained constant, or, alternatively, an actual upward or downward trend in CVP may be masked by the change in measured CVP. Accordingly, consistency in aligning the pressure monitoring device in relation to the right atrium over time is perhaps just as important, if not more important, than accurately aligning the pressure monitoring device with the right atrium initially.

The presently disclosed device supporting one or more pressure measuring devices can be consistently and accurately aligned with the right atrium of the heart so that actual CVP and upward and downward trends in CVP are accurately monitored as further discussed below.

The present application also provides a clip with an alignment device (e.g., an alignment laser) to facilitate the appropriate placement of a transducer (e.g., a reusable or a disposable pressure transducer) at the phlebostatic axis (i.e., the approximate location of the right atrium, which is found at the intersection of the midaxillary and a line drawn from the fourth intercostal space at the right side of the sternum) for zeroing. The alignment device may be coupled (e.g., removably coupled) to clip without occupying a slot or connector on an outer surface of an arm otherwise useable for other medical devices themselves.

The present application also provides for mounting on multiple pole sizes, on a vertical pole, and on a pole angled from a vertical axis by 45 degrees. To accomplish this, the clip may include rotatable and/or static structures for mounting the clip on a pole or other object. The clip may include structures configured for mounting the clip on objects of different sizes, such as pole of different diameters.

The presently disclosed clip provides simplicity and convenience to the user by providing ease of adjustment, relieving space on the clip for other components otherwise used by an alignment device, the ability to quickly attach to multiple pole sizes and may allow single-hand adjustment of the clip. The presently disclosed clip also provides for mounting on poles or other objects that are not vertical, but may still facilitate maintaining the vertical alignment of transducers on the clip with a patient (e.g., with the right atrium of the patient's heart).

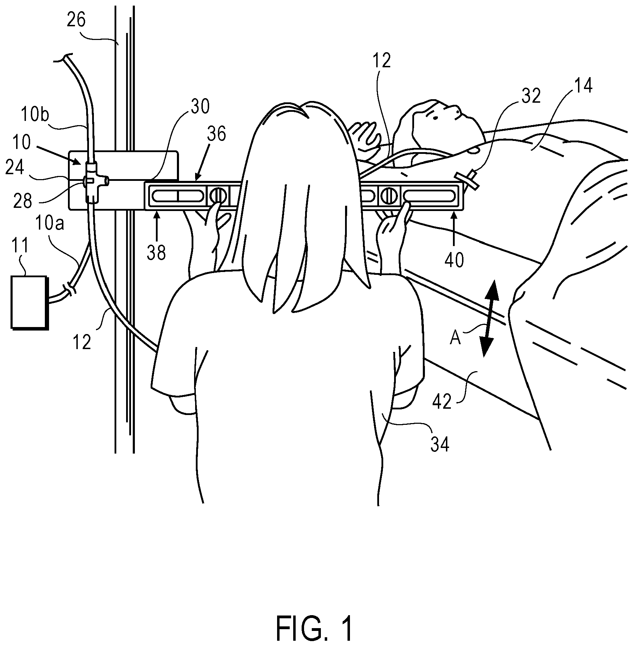

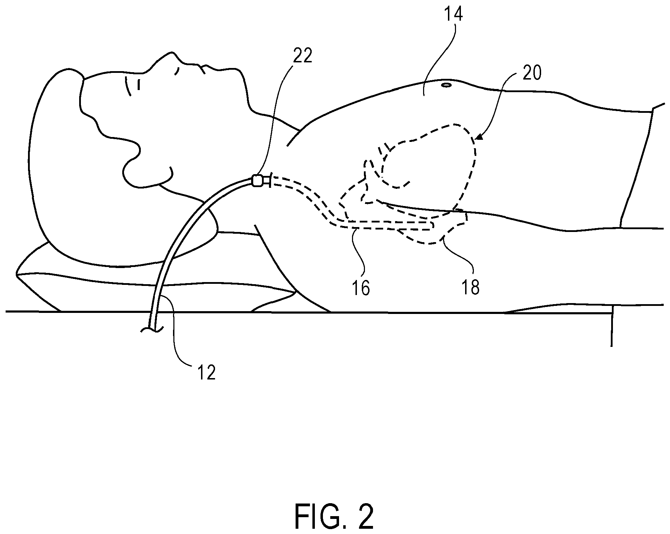

Depicted in Figure 1 is a conventional method of measuring blood pressure in the right atrium of the heart. In Figure 1, the pressure monitoring device is a transducer 10. A length of IV connecting tubing 12 extends from a patient 14 to the transducer 10. As depicted in Figure 2, a catheter 16 has been inserted into the patient's subclavian vein and threaded through this vein into the right atrium 18 of the patient's heart 20. The catheter 16 is connected at 22 to the IV connecting tubing 12. The catheter 16 and IV tubing 12 are filled with fluid; thus, the pressure of blood in the right atrium 18 is transmitted to the fluid within the catheter 16 and tubing 12 where this pressure can be measured by the transducer 10.

The transducer 10 is attached to a mounting bracket 24 which is slideably mounted on a vertically extending pole 26. The vertical level of a given measuring point 28 of the transducer 10 is indicated by a line 30 formed on the bracket 24. The measuring point 28 is at the balancing port of the transducer 10.

The transducer 10 further includes a cable 10a through which electrical signals related to sensed pressure are transmitted to an analyzing device 11 that calculates and records CVP based on these electrical signals. A second length of IV connecting tubing 10b extends from the top of the transducer 10 to container (not shown) containing an IV solution to be introduced into the patient 14. This allows fluid within the tubing 12 to be flushed and to allow introduction of intravenous fluids and medications into the patient's bloodstream as necessary.

The vertical level of the right atrium 18 of the patient's heart 20 generally corresponds to the midaxillary line of the patient 14 and is marked by an X-mark 32 formed on the patient 14 approximately 5 cm below the patient's sternum. While this X-mark may not be precisely aligned with the patient's right atrium in all cases, the X-mark is generally formed on the patient with indelible ink so that measurements may at least be consistent over time.

To align the measuring point 28 of the transducer 10 with the end of the catheter 16 located within the right atrium 18, a health care worker 34 uses a leveling device such as a conventional three-foot carpenter's level 36. Alternatively, the leveling device may be a length of rigid material such as a wooden yardstick having a known bubble indicator attached to the center portion thereof to indicate when the yardstick is horizontal.

A first end 38 of the carpenter's level 36 is initially placed along the line 30 formed on bracket 24. The health care worker 34 then raises and lowers the second end 40 of the level 36 until the horizontal bubble indicator (not shown) on the level 36 indicates that the level 36 is horizontally aligned. When the level 36 is horizontally aligned, the worker 34 determines whether the second end 40 of the level 36 is aligned with the X-mark 32.

If the second end 40 is not aligned with the X-mark 32, the worker 34 must raise or lower as appropriate, in the direction indicated by the arrow A, either the patient 14 by raising and lowering the patient's bed 42 or the transducer 10 by raising and lowering the bracket 24 on which the transducer 10 is mounted. In either case, the worker 34 cannot continue to hold the level 36 such that it is vertically aligned because to do so requires two hands and at least one hand, and preferably two hands, must be free to adjust the bed 42 or bracket 24. The health care worker 34 must therefore set the level 36 down, adjust the bed 42 or bracket 24, and then raise and horizontally align the level 36 to check whether the X-mark 32 is properly aligned with the second end 40. This process is repeated until the second end 40 of the horizontally-aligned level 36 is vertically aligned with the X-mark 32.

One problem with the conventional method shown in Figure 1 is that this method may be difficult to perform quickly and accurately, especially when performed by one person. This is because it may be difficult to hold the level 36 in its horizontally aligned position while determining whether the second end 40 thereof is aligned with the X-mark 32.

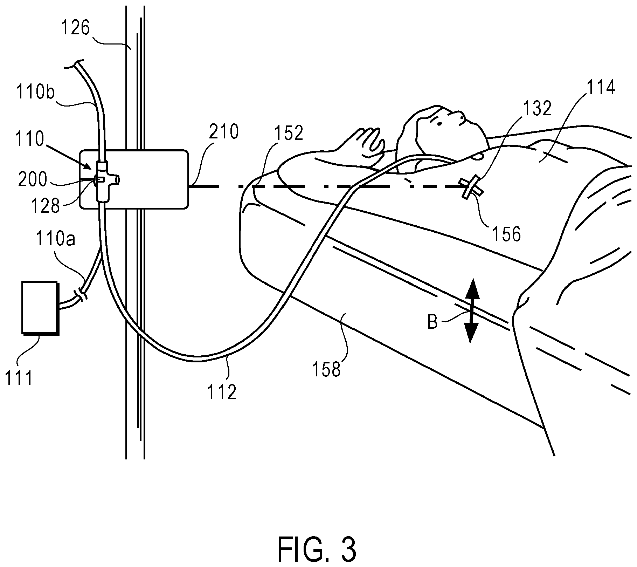

To address these problems, the present invention embraces a clip having an alignment device that emits an alignment signal. In this regard, Figure 3 depicts a transducer 110 (e.g., a transducer module) attached to a clip 200 having an alignment device 210 in accordance with the present invention. As depicted in Figure 3, the clip 200 may be attached to a vertically extending pole 126. The alignment device 210 typically includes a housing, a light source, and an aperture through which a beam of light, emanating from the light source, extends along a path. Although the alignment device 210 typically emits a visible alignment signal (e.g., a beam of visible light), in some embodiments the alignment signal may not be visible, such as a beam of non-visible electromagnetic radiation (e.g., ultraviolet or infrared radiation). The alignment device 210 is typically attached to the clip 200 so that the alignment device 210 is at the same vertical level as the transducer's measurement point 128 (e.g., located at a stopcock valve in the module).

As depicted in Figure 3, a length of outgoing IV tubing 112 extends from a patient 114 to the transducer 110. In addition, a catheter has been inserted into the patient's subclavian vein and threaded through this vein into the right atrium of the patient's heart. The catheter is connected to the outgoing IV tubing 112. The catheter and outgoing IV tubing 112 are filled with fluid; thus, the pressure of blood in the right atrium is transmitted to the fluid within the catheter and outgoing IV tubing 112 where the pressure can easily be measured by the transducer 110.

As depicted in Figure 3, the transducer 110 further includes a cable 110a through which electrical signals related to sensed pressure may be transmitted to an analyzing device 111 that calculates and records CVP based on these electrical signals. The analyzing device 111 may be, for example, a CVP module designed to be plugged into computer monitor or monitoring system. In addition, Figure 3 depicts a second length of incoming IV tubing 110b that extends from the top of the transducer 110 to a container (not shown) that may contain an IV solution to be introduced into the patient 114. This allows fluid within the outgoing IV tubing 112 to be flushed and allows introduction of intravenous fluids and medications into the patient's bloodstream as necessary.

The approximate location of the right atrium of the patient's heart generally corresponds to the phlebostatic axis of the patient 114 and can be marked by an optional indicia (e.g., an X-mark 132) formed on the patient 114, typically on a region approximately 5 cm below the patient's sternum. The X-mark can be provided on the patient with indelible ink so that measurements may at least be consistent over time.

The clip 200 is configured to facilitate the alignment one or more transducers 110 (e.g., a pressure transducer module) attached to the clip 200 with the X-mark 132, to thereby align the transducers 110 with the approximate location of the right atrium of the patient's heart (e.g., the phlebostatic axis of the patient). Accordingly, the clip 200 includes an alignment device 210.

In order to align the transducer 110 with the X-mark 132, first the clip 200 is mounted on the pole 126. Thereafter, the transducer 110 is mounted on the clip 200. In addition, the transducer 110 is attached to the analyzing device 111 through the cable 110a. The outgoing IV tubing 112 is then connected between the transducer 110 and the catheter. Next, light is emitted along the light path 152 from the alignment device 210 to cause a light indicia 156 (e.g., a light indicia created by a narrow ray or beam of light) to visually appear on the patient 114. In this regard, a light source (e.g., laser) included in the alignment device may be turned on. The light source may remain on until turned off by a user, or may automatically turn off after a predetermined period of time. Thereafter the clip, pole, and/or patient are moved until the light indicia 156 is located at the X-mark 132 in order to vertically align the transducer's measurement point 128 with the approximate location of the right atrium of the patient's heart. For example, the clip may be horizontally rotated around the pole or vertically moved along the pole. As further described herein, to increase the accuracy of any measurements from the transducer, a user may also ensure that the alignment device 210 and/or the clip 200 are horizontally level (e.g., using a bubble level attached to the clip 200).

The transducer 110 and analyzing device 111 may then be used to accurately and consistently measure and record the CVP of the patient 114. In this manner, a single health care worker can easily and quickly align the measuring point 128 with the X-mark 132. As the light indicia 156 is continually shining on the patient 114 during the process of adjusting the height of the bed 158 or the clip 200, the present clip 200 significantly reduces the chances that the transducer measuring point 128 will be inaccurately aligned with the X-mark 132.

In some instances, a sensor for detecting the presence of the light indicia 156 may be placed on the patient (e.g., on the X-mark 132) to facilitate alignment of the clip 200. Such sensor may be in communication with a device (e.g., computer, mobile device, or the like) that provides an indication (e.g., a visual or audio indication) of whether the light indicia 156 is located at the light sensor. If the alignment device does not emit visible light but instead emits non-visible electromagnetic radiation, the sensor may be able to detect whether a beam of electromagnetic radiation is directed towards the sensor. Once the clip 200 has been aligned, the device may also provide an indication if clip 200 later becomes out of alignment (e.g., by determining that the light indicia 156 is no longer located at the light sensor).

Although the alignment device 210 of the clip 200 is described herein as being used to align the clip 200 and any attached transducers with the right atrium of a patient's heart, it is within the scope of the present invention to similarly employ the alignment device 210 to align the clip 200 and any attached medical device with any point on a patient.

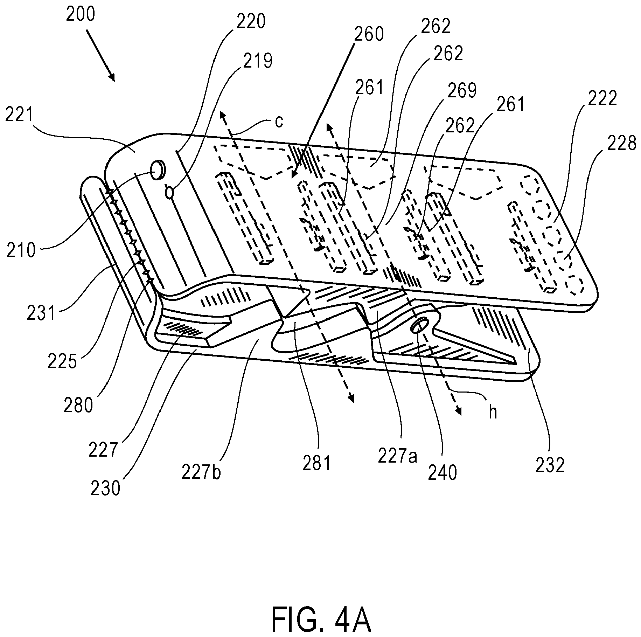

Figures 4A-4B schematically illustrates an exemplary embodiment the clip 200 in more detail. As depicted in Figures 4A-4C, the clip 200 includes a body formed by the first and second arms 220 and 230 (e.g., elongated arms). The first and second arms 220, 230 are typically formed from a rigid material (e.g., a rigid plastic). Connectors 260 are positioned along the body and each is configured to releasably connect with a transducer 110. The clip 200 is movable between first and second orientations, typically open and closed orientations, to attach to an object that is placed within one or more attachment locations 280, 281 formed within the body. The first and second attachment locations 280, 281 may include different configurations to provide for attaching to different types of objects (e.g., a pole, a bed post, a bed sheet, an article of the patients clothing, a curtain, and the like).

As illustrated in Figures 4A-4C, the first arm 220 and second arm 230 are pivotally mounted together at a hinge 240. Each of the arms 220, 230 may include a compound shape with first ends 221, 231 curved inward towards each other and second ends 222, 232 curved outward away from each other. In one particular embodiment, the length of each of the arms 220, 230 measured between the first and second ends 221, 222, 231, 232 is about 15.24 cm (6 inches) the width (perpendicular to the length) is about 6.35 cm (2.5 inches), and the thickness of the arms 220, 230 is about 2.54 cm (1 inch). The arms 220, 230 may include the same or different dimensions. Each arm typically defines a longitudinal axis *l* between its first and second ends.

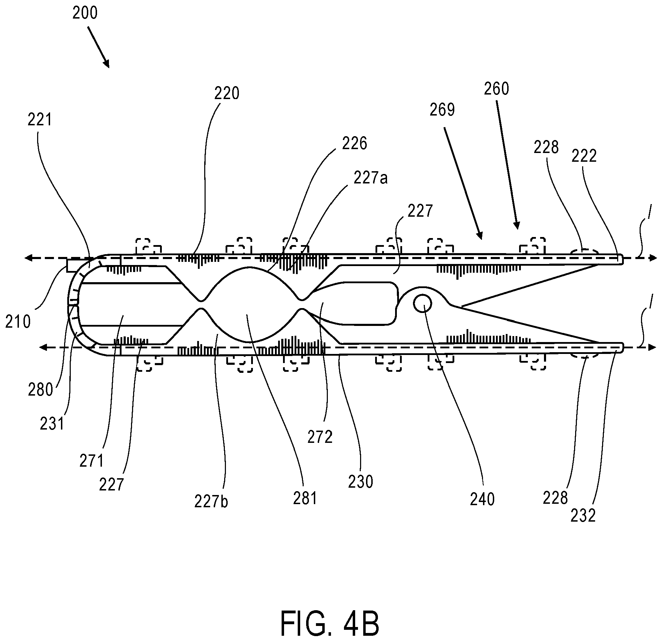

The first attachment location 280 is generally formed at the first end of the arms 220, 230 and is typically configured to attach the clip 200 to a first type of object, typically a flexible object, such as a bed or clothing. Teeth 225 may be formed on one or both of the first ends 221, 231 to facilitate attachment when an object is positioned in the first attachment location 280. The second attachment location 281 is generally spaced inward from the first ends of the arms 220, 230 and is typically sized and configured to attach the clip 200 to a second object, typically a cylindrical object, such as a pole. The clip 200 is typically positionable between an open orientation with first ends 221, 231 of the arms 220, 230 spaced a first distance apart, and a closed orientation with the first ends 221, 231 a lesser distance apart and either touching (e.g., as depicted in Figure 4B) or in close proximity for attachment to an object.

Ribs 227 may extend along the inner sides of the arms 220, 230. In one embodiment, a pair of ribs 227 extend along the length of each arm 220, 230. The ribs 227 may extend an entirety or limited distance along the length of the arms 220, 230. The pair of ribs 227 on each arm 220, 230 may be parallel. The pair of ribs 227 are further spaced-apart across the width of each arm 220, 230 with a space formed between the ribs. The ribs 227 on each arm 220, 230 may have an identical shape and size.

The ribs 227 on the first arm 220 may overlap with the ribs 227 on the second arm 230. Such overlap may provide for forming a pivoting connection between the arms 220, 230. In this regard, each of the ribs 227 may include an opening that together align and are sized to receive the hinge 240 to pivotally connect the first and second arms 220, 230. In one embodiment, the ribs 227 of the first arm 220 are positioned in a side-by-side arrangement with the ribs 227 of the second arm 230.

As illustrated in Figure 4B, the ribs 227 may be shaped and sized to form openings 271, 272. As illustrated in Figure 4B, the first opening 271 is adjacent to and positioned on a first side of the second attachment location 281 and the second opening 272 is adjacent to and positioned on an opposing second side of the second attachment location 281. One or both of the openings 271, 272 may include a tapered shape with a width that decreases towards the second attachment location 281.

The ribs 227 may be attached to first and second structures 227a, 227b that form at least a portion of the second attachment location 281 (e.g., by forming a compartment between the first and second structures 227a, 227b). In one embodiment, each of the first and second structures 227a, 227b includes an indentation 226 sized to extend around a portion of the inserted object (e.g., a pole). In one embodiment as illustrated in Figure 4B, each indentation 226 includes a rounded shape. In one embodiment, the indentations 226 of the opposing first and second structures 227a, 227b form a perimeter (e.g., a perimeter that completely or substantially surrounds an inserted object) when the clip 200 is in the closed orientation. The indentations 226 may also have a variety of other shapes and sizes (e.g., forming a circular or elliptical cross-sectional shape when the clip 200 is in the closed orientation). The indentations 226 are typically positioned about the same common distance away from the first ends 221, 231 of their respective arm 220, 230 to align together.

Additional supports may extend between the ribs 227 of each of the arms 220, 230 and form a portion of the second attachment location 281. In one embodiment, additional supports may include partial cylinders that align together in the closed orientation such that the second attachment location 281 includes a cylinder along much of the width of the clip 200, instead of more limited attachment locations. As depicted in Figure 4A, a substantially cylindrical-shape formed by the second attachment location 281 in the closed orientation may define an axis c that is substantially parallel with the hinge's axis of rotation *h.*

The hinge 240 typically extends through the first and second arms 220, 230 and forms a pivoting axis (e.g., for transitioning the arms 220, 230 between the first and second orientations). The hinge 240 may be a single elongated member that extends through the ribs 227 on each of the first and second arms 220, 230. The hinge 240 may also include two or more separate members that extend through a portion of each of the first and second arms 220, 230. In one embodiment, a first hinge portion extends through a first pair of ribs 227 on the first and second arms 220, 230, and a second hinge portion extends through a second pair of ribs 227. In another embodiment, each of the pairs of ribs includes a ball-and-detent configuration to attach the arms 220, 230 in a pivoting arrangement.

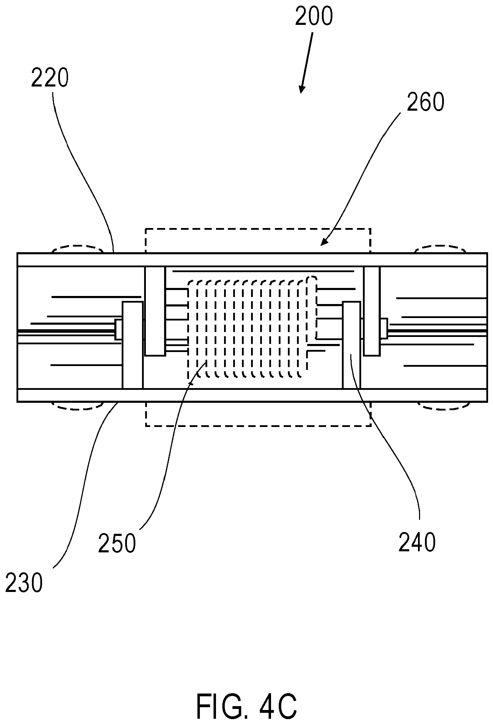

A biasing member 250 (e.g., a spring) is typically positioned between the arms 220, 230 to bias the clip 200 towards a closed orientation. The biasing member 250 typically applies a biasing force to the arms 220, 230 to separate the second ends 222, 232 and force the first ends 221, 231 together. In some embodiments, the biasing member 250 may be attached to the arms 220, 230 at a point in front of the hinge 240 (i.e., between the hinge 240 and first ends 221, 231) and apply an inward force to bias the arms 220, 230 towards the closed orientation. In one embodiment as illustrated in Figure 4C, the biasing member 250 may include a spring that wraps around the hinge 240. In another embodiment, the biasing member 250 is positioned away from the hinge 240 and directly contacts against the inner surfaces of the arms 220, 230 at a point in proximity to the second ends 222, 232. The clip 200 may use various other types of biasing members 250, including a flexible material positioned between the arms 220, 230 towards the second ends 222, 232.

In use, the clip 200 is typically biased towards the closed orientation. To attach the clip 200 to an object, an inward force is typically applied to the back portion of the arms 220, 230 at a point between the hinge 240 and the second ends 222, 232. To facilitate application of the inward force, the outer surfaces of one or both of the arms 220, 230 may include a contact surface or grip 228. As depicted in Figures 4A-4B, the grip 228 may include a plurality of elevated nodes that are spaced across the width of the clip 200. This inward force moves the clip 200 to the open position with the first ends 221, 231 spaced apart.

In the open orientation, the clip 200 can be attached to different types of objects. One type of object can be gripped within the first attachment location 280 formed between the first ends 221, 231. This may include positioning clothing, a sideboard of a bed, and bedding (e.g., blankets, sheets, etc.) in the first attachment location 280. The teeth 225 may facilitate such attachment. Once positioned on the object, the arms 220, 230 may be released resulting in the biasing member 250 forcing the first ends 221, 231 of the arms 220, 230 together towards the closed orientation. This force is typically adequate to attach the clip 200 to the object. In this regard, the characteristics (e.g., size, exerted force, and the like) of the biasing member 250 may be adjusted depending on the size of the object and the force needed to adequately grip the object.

The clip 200 is also configured to attach to an object at the second attachment location 281. In this type of use, the object (e.g., a pole have a cylindrical shape) may inserted into the space formed between the spaced apart arms 220, 230 when the clip is in the open orientation. The object may be aligned in the second attachment location 281 and the arms 220, 230 are released causing the clip 200 to move towards the closed orientation.

One or more of the connectors 260 are typically positioned on an outer side (i.e., exterior side) of one or both arms 220, 230. Each connector 260 is typically configured to receive a transducer 110 (e.g., an arterial blood pressure transducer module). The connectors 260 are typically spaced-apart across the length of outer side of the clip 200 to provide adequate spacing to receive each of the transducers 110. Typically, the connectors 260 are further oriented to direct the incoming tubing 110b, the outgoing IV tubing 112, and the cable 110a away from the first ends 221, 231 and second ends 222, 232 of the clip 200 where they could possible by damaged during movement of the clip 200 between the open and closed orientations. Additional connectors 260 may be positioned along the outer side of the opposing arm to receive additional transducers 110. Figures 4A-4B include three connectors 260 positioned on the outer surfaces of each of the arms 220, 230. Other embodiments may include more or fewer connectors 260 on the arms 220, 230. The arms 220, 230 may include the same or different numbers of connectors 260. In embodiments with multiple connectors 260, the connectors 260 may be spaced at various locations along the length of the arms 220, 230 between the first and second ends 221, 222, 231, 232.

As depicted in Figure 4A, each connector 260 typically includes a pair of opposing sidewalls 261 that form a channel 269 for receiving a transducer 110. The channels 269 are aligned to direct the incoming and outgoing tubing and the cable of the transducers 110 away from the first and second ends 221, 231, 222, 232. Protrusions 262 extend outward and are positioned between the sidewalls 261 to align and position the transducer 110 in the channel 269. Each connector 260 may further include a label 263 for identifying the specific transducer.

In other embodiment, the connectors 260 include various other shapes, sizes, and configurations. In this regard, the connectors 260 may be configured to receive transducers 110 from a variety of different sources, including Baxter, ICU Medical, Hospira, and Edwards Life Sciences.

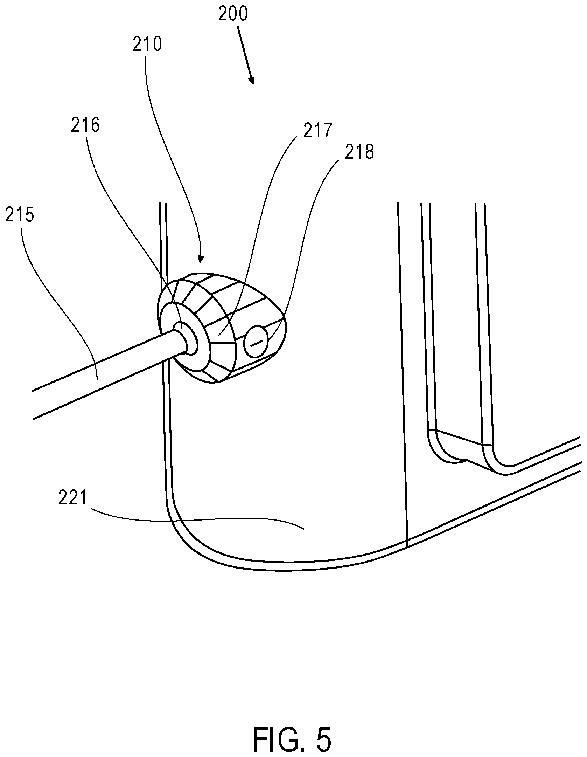

As noted, the clip 200 includes an alignment device 210 that is configured to emit an alignment signal. This alignment signal is a light beam that causes a light indicia to visually appear on a patient. That said, in some embodiments the alignment signal may not be visible, such as a beam of non-visible electromagnetic radiation (e.g., ultraviolet or infrared radiation). As depicted in Figures 4A-4B and 5, the alignment device 210 may be attached to the first end of one of the arms 220, 230 (e.g., the first end 221 of the first arm 220). That said, the alignment device may be attached to other portions of the clip 200 (e.g., near the second end of one of the arms). The alignment device 210 may be fixedly attached to the clip 200 (e.g., via an adhesive or one or more screws) or removably attached to the clip 200 (e.g., via a snap fastener). If the alignment device 210 is configured to be removably attached to the clip 200, the clip 200 may include multiple attachment locations 219 to which the alignment device 210 may be attached (e.g., as depicted in Figure 4A). As noted, the alignment device 210 is typically attached to the clip 200 so that the alignment device 210 is aligned with the measuring point 128 of the transducer(s) 110. Some transducers may have differing measuring points. As such, if the clip 200 includes multiple attachment locations 219, each attachment location 219 may be aligned with the measuring point associated with a particular type of transducer.

Figure 5 depicts an exemplary alignment device 210 in accordance with a particular embodiment of the present invention. As depicted in Figure 5, the alignment device 210 includes a light emitting device 216 (e.g., a laser) positioned within a device housing 217. While the light emitting device 216 typically includes a laser (e.g., a light emitting diode (LED) laser) that generates coherent electromagnetic radiation, a non-coherent light source may be used with appropriate lenses for focusing the light beam thereof to produce a light spot of sufficiently small size for the purposes of the present disclosure. The alignment device 210 may include an actuator 218 (e.g., a button) that may be used to turn the light emitting device 216 on or off. In some embodiments, the light emitting device 216 may remain on until turned off by a user. In other embodiments, the light emitting device 216 may automatically turn off after a predetermined period of time. When turned on, the light emitting device 216 emits a light beam 215, which can then be used to align the clip 200 with a patient. In this regard, the alignment device 210 may be configured so that the light beam 215 emitted is substantially parallel to the longitudinal axis of the arm to which the alignment device 210 is attached.

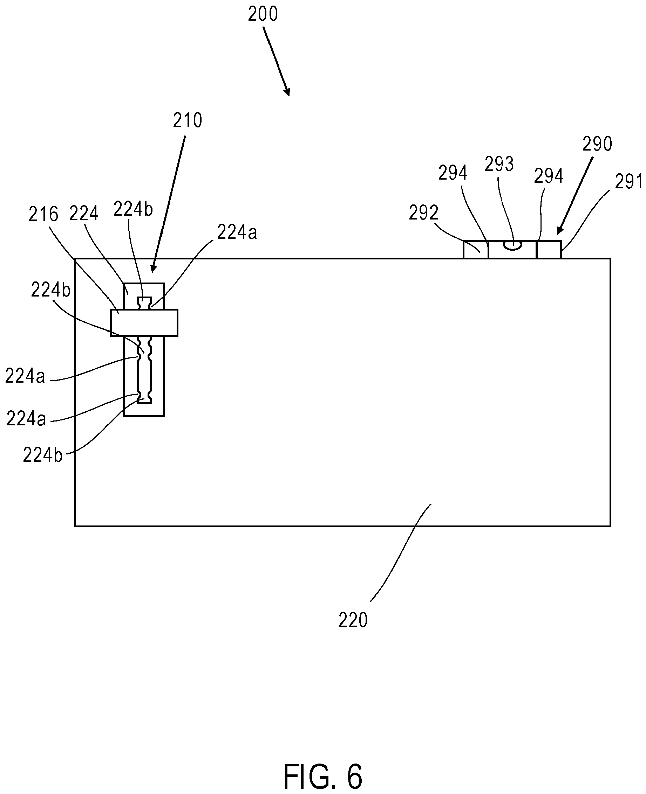

In one embodiment, the light emitting device 216 may be movable along a portion of the width of the clip 200. For example, as depicted in Figure 6, the alignment device 210 may include a rail 224 running along at least a portion of the width of the clip 200. The light emitting device 216 may be movable along the rail 224 so that the light emitting device 216 can be aligned with the measuring points of different types of transducers. In this regard, the rail may include a plurality of light emitting device locations 224b that correspond to the measuring points of different types of transducers. The rail 224 may further include protrusions 224a for maintaining the position of the light emitting device 216 at one of the light emitting device locations 224b.

As also shown in Figure 6, the clip 200 includes a bubble level 290 attached to one of the arms that a user can employ to ensure that the clip 200-and in some instances the light beam-is horizontally level. As shown in Figure 6, the bubble level 290 typically includes a closed glass envelope 291 containing a fluid 292 and a bubble 293 of air or other gas. Two lines 294 are marked on the envelope 291. When a longitudinal axis of the bubble level 290, and thus the clip 200, is horizontally aligned, the bubble 293 is between the lines 294. By ensuring that (i) both the clip 200 and the light beam are horizontally level and (ii) the light indicia appears at the correct location on a patient (e.g., on the X-mark 132), a user ensures that the measuring points of any attached transducers are vertically aligned with the phlebostatic axis of the patient.

The alignment device is rotatable relative to the clip 200. In particular the light emitting device 216 and device housing 217 are rotatable ( about an axis orthogonal to the arm to which the alignment device 210 is attached). In some particular embodiments, the alignment device 210 may include a self-leveling feature to ensure that a rotatable light emitting device 216 or a rotatable optical element maintains a horizontal alignment (e.g., to ensure that the alignment device 210 emits a horizontal light beam regardless of the orientation of the clip 200). In alternative embodiments, the alignment device 210 may be frictionally engaged with an arm so that a minimum torque must be applied to cause the alignment device 210 to rotate relative to the clip 200. This minimum torque is typically greater than the torque applied by the weight of the alignment device 210 plus a safety factor. As such, in some embodiments the alignment device 210 may not rotate relative to the clip 200 without user intervention. In further embodiments, the alignment device 210 might be rotatable in only one direction. For example, the alignment device 210 might be engaged to an arm in a gear and pawl arrangement. In some embodiments, the alignment device 210 might be freely rotatable about an axis (e.g., able to rotate 360 degrees); however, in other embodiments, the angle of rotation may be limited (e.g., to 90 degrees).

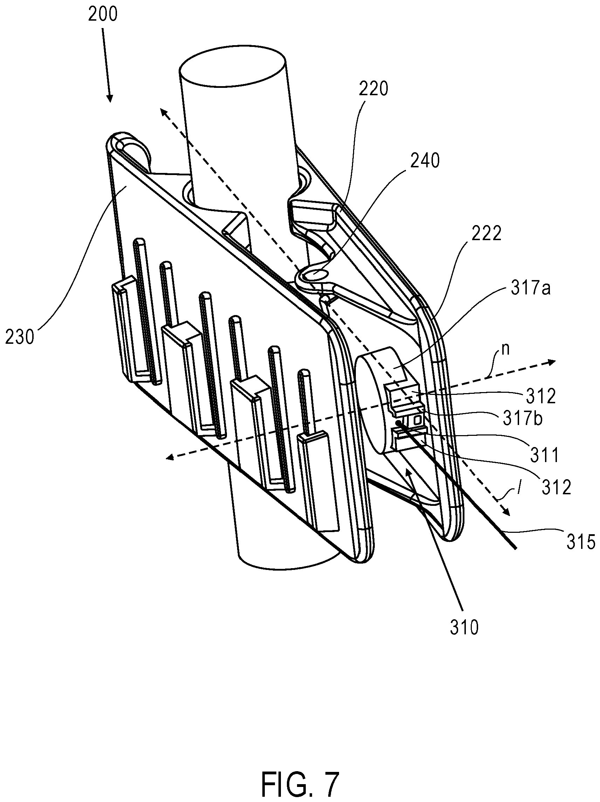

By way of example, Figure 7 depicts an exemplary alignment device 310 that has a rotatable housing portion 317a that is configured to rotate about an axis *n* orthogonal to the longitudinal axis *l* of the first arm 220 (or orthogonal to a plane that includes to the longitudinal axis *l* of the first arm 220). The rotatable housing portion 317a is rotatably coupled to a fixed (i.e., not rotatable relative to the clip) housing portion 317b. The fixed housing portion 317b may be fixedly or removably attached to the first arm 220 (e.g., near the second end 222 of the first arm 220). The alignment device 310 includes a light emitting device. The rotatable housing portion 317a is configured to direct a light beam from the light emitting device such that the direction of the light beam rotates with the rotatable housing portion 317a (e.g., about an axis orthogonal to the first arm 220). In a particular embodiment, the light emitting device may rotate with the rotatable housing portion 317a. The rotatable housing portion 317a may include a self-leveling feature to ensure that the alignment device 210 emits a horizontal light beam regardless of the orientation of the clip 200. In some embodiments, the alignment device 310, may include an aperture 311 that allows the passage of light directed from the rotatable housing portion 317a at narrow range of angles. In addition, the aperture 311 may include blocking portions 312 that obstruct light directed from the rotatable housing portion 317a outside such narrow range of angles. In this regard, as depicted in Figure 7, the aperture 311 is typically positioned such that a light beam 315 directed from the rotatable housing portion 317a must be substantially parallel to the longitudinal axis *l* of the first arm 220 in order pass through the aperture; otherwise the light beam may be blocked (e.g., by the blocking portions 312). The aperture 311 in combination with the rotatable housing portion 317a being self-leveling can be used to ensure that both the clip 200 and the light beam 315 are horizontally level. By ensuring that (i) both the clip 200 and the light beam 315 are horizontally level and (ii) the light indicia appears at the correct location on a patient (e.g., on the X-mark 132), a user can ensure that the measuring points of any attached transducers are vertically aligned (e.g., in the same horizontal plane) with the phlebostatic axis of the patient.

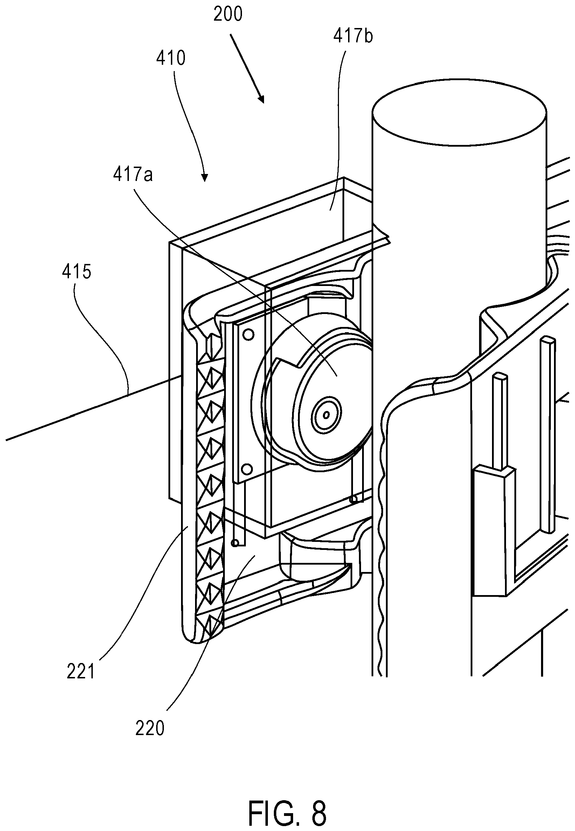

By way of further example, Figure 8 depicts another exemplary alignment device 410 that has a rotatable housing portion 417a that is configured to rotate about an axis orthogonal to the first arm 220 (or orthogonal to a plane that includes to the longitudinal axis of the first arm 220). The rotatable housing portion 417a is rotatably coupled to a fixed (i.e., not rotatable relative to the clip) housing portion 417b. The fixed housing portion 417b may be fixedly or removably attached to the first arm 220 (e.g., near the first end 221 of the first arm 220). The alignment device 410 includes a light emitting device. The rotatable housing portion 417a is configured to direct a light beam 415 from the light emitting device such that the direction of the light beam rotates with the rotatable housing portion 417a (e.g., about an axis orthogonal to the first arm 220). In a particular embodiment, the light emitting device may rotate with the rotatable housing portion 417a. The rotatable housing portion 417a may include a self-leveling feature to ensure that the alignment device 210 emits a horizontal light beam regardless of the orientation of the clip 200. By ensuring that the light beam 415 is horizontally level regardless of the orientation of the clip 200, a user can approximately vertically align the measuring points of any attached transducers with a patient (e.g., so that the measuring points and the phlebostatic axis of the patient are in the same horizontal plane).

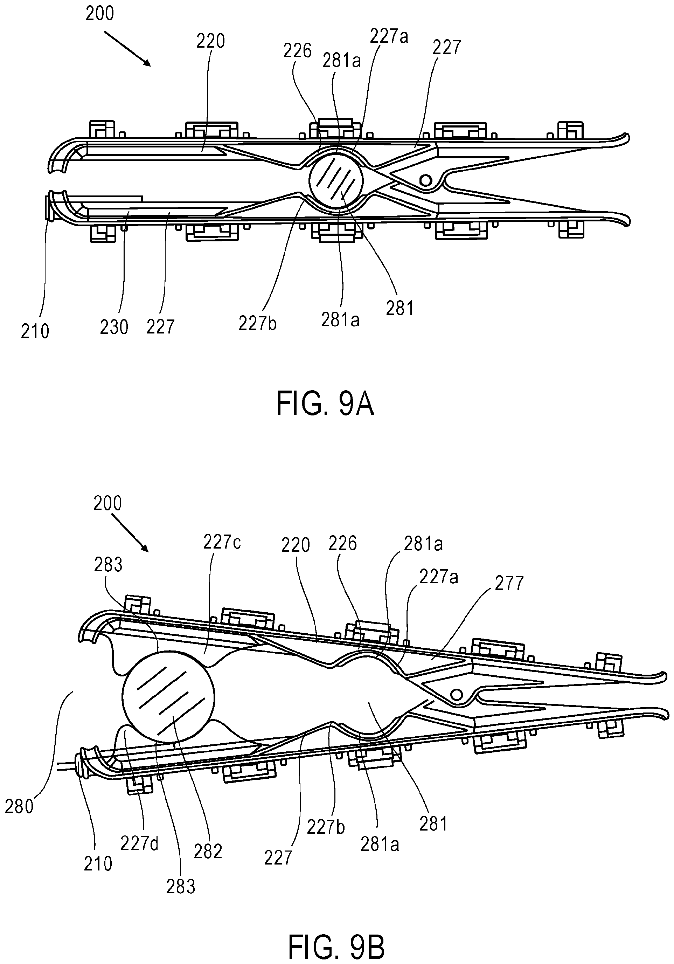

In some instances, it may be desirable for the clip 200 to be attachable to objects of different sizes (e.g., poles having different diameters). That said, when the second attachment location 281 has a cylindrical shape, the size range of objects that can be attached to the second attachment location 281 may be limited. As depicted in Figure 9A, to increase the range of objects sizes that can be engaged by the second attachment location 281, the second attachment location may include flexible grips 281a attached to the first and second structures 227a, 227b that form the second attachment location 281. To facilitate attachment of the clip 200 to objects of varying sizes (e.g., diameters), the flexible grips 281a are typically formed from a flexible and compressible material.

To facilitate attachment of the clip 200 to larger objects (e.g., poles having larger diameters) the clip may include a third attachment location 282. As illustrated in Figure 9B, the third attachment location 283 may be positioned between the first attachment location 280 and the second attachment location 281. In this regard, the ribs 227 may be attached to third and fourth structures 227c, 227d that form at least a portion of the third attachment location 282 (e.g., by forming a compartment between the third and fourth structures 227c, 227d). In one embodiment, each of the third and fourth structures 227c, 227d includes an indentation 283 sized to extend around a portion of the inserted object (e.g., a pole). As illustrated in Figure 9B, each indentation 283 typically includes a rounded shape. In combination, these indentations 283 typically form a substantially cylindrical-shaped third attachment location 282 (e.g., when the clip is in the closed position). Similar to the second attachment location 281, this cylindrical-shaped third attachment location may define an axis that is substantially parallel with the hinge's axis of rotation. To increase the range of objects sizes that can be engaged by the third attachment location 282, the third attachment location 282 may include flexible grips attached to the third and fourth structures 227c, 227d that form the third attachment location 282. To facilitate attachment of the clip 200 to objects of varying sizes (e.g., diameters) at the third attachment location 282, such flexible grips may be formed from a flexible and compressible material.

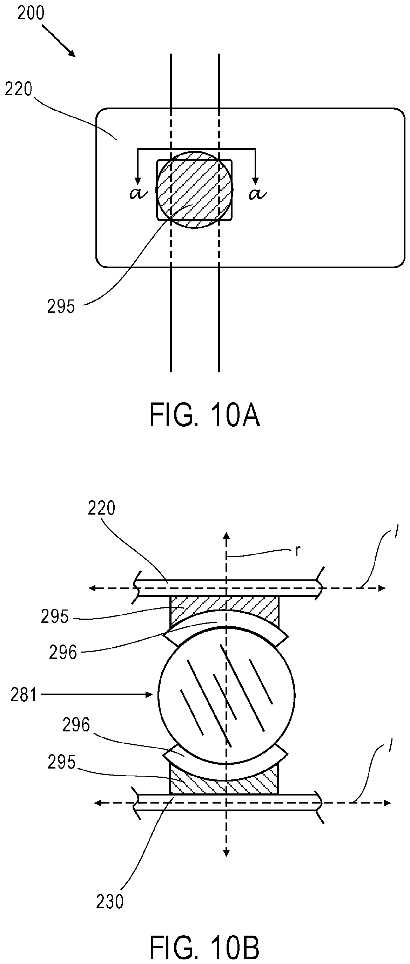

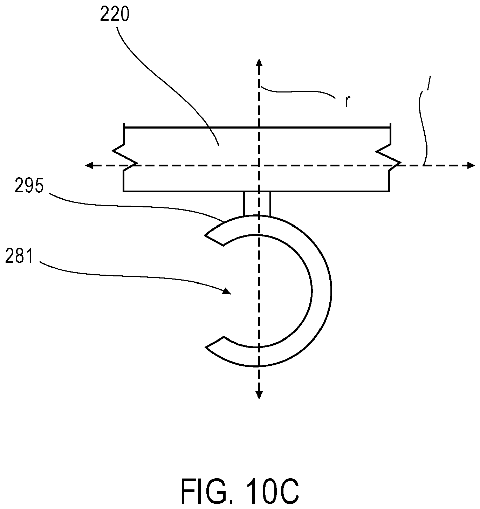

In some instances it may be desirable to attach the clip 200 to an object, such as a pole, that is not vertically aligned. For example, a pole may be positioned at a 45 degree angle relative to the ground rather than at a 90 degree angle relative to the ground. That said, it may still be desirable to maintain the clip 200 as being horizontally level. Accordingly, in contrast with Figures 4A-4B which depict static (i.e., not rotatable) attachment locations, one or more of the attachment locations (e.g., the second attachment location 281) may be rotatable relative to the clip 200. In this regard, Figures 10A-10B depict the second attachment location 281 being rotatable relative to the clip 200. As depicted in Figures 10A-10B, the attachment location 281 may be formed from a rotatable structure 295 located on (e.g., attached to) each arm 220, 230. Together, the rotatable structures 295 typically form the attachment location 281 such that the attachment location 281 has a substantially cylindrical-shaped structure when the clip 200 is in the closed position as depicted in Figure 10B. Each rotatable structure 295 may further include a flexible grip 296 (e.g., a rubber grip) to facilitate attachment of an object within the second attachment location 281. Each rotatable structure 295 is typically rotatable about an axis *r* that is orthogonal to the longitudinal axis *l* of the arm to which each rotatable structure 295 is attached (or orthogonal to a plane that includes to the longitudinal axis *l* of the arm to which each rotatable structure 295 is attached). As depicted in Figure 10B, when the longitudinal axes *l* of the arms 220, 230 are parallel to one another, and the rotatable structures 295 are in a fixed position (e.g., attached to a fixed object, such as a pole), the first and the second arms 220, 230 are rotatable about the axis r that is substantially orthogonal to two substantially parallel planes that each includes one of the longitudinal axes *l* of the first and the second arms 220, 230. To facilitate such rotation, each rotatable structure 295 may be attached to an arm via a revolute joint. Each rotatable structure 295 may be frictionally engaged with its corresponding arm so that a minimum torque must be applied to cause the rotatable structures 295 to rotate relative to the clip 200. This minimum torque is typically greater than the torque applied by the maximum expected weight of the clip 200 (e.g., the expected weight of the clip 200 when a transducer is attached to each connector 260) plus a safety factor. As such, in some embodiments the clip 200 may not rotate relative to the rotatable structures 295 without user intervention. In some embodiments, each rotatable structure 295 might be rotatable in only one direction. For example, each rotatable structure 295 might be engaged to its corresponding arm in a gear and pawl arrangement. Although the attachment location 281 may be formed from two rotatable structures 295, each rotatable structure 295 being rotatably coupled to an arm of the clip 200, in an alternative embodiment depicted in Figure 10C, the attachment location 281 may be formed from a single rotatable structure 295 that is attached to one arm of the clip 200. The rotatable structure 295 depicted in Figure 10C (i) typically has a cylindrical shape and (*ii*) typically is rotatable about an axis *r* orthogonal to a plane that includes the longitudinal axis *l* of arm to which the rotatable structure 295 is attached.

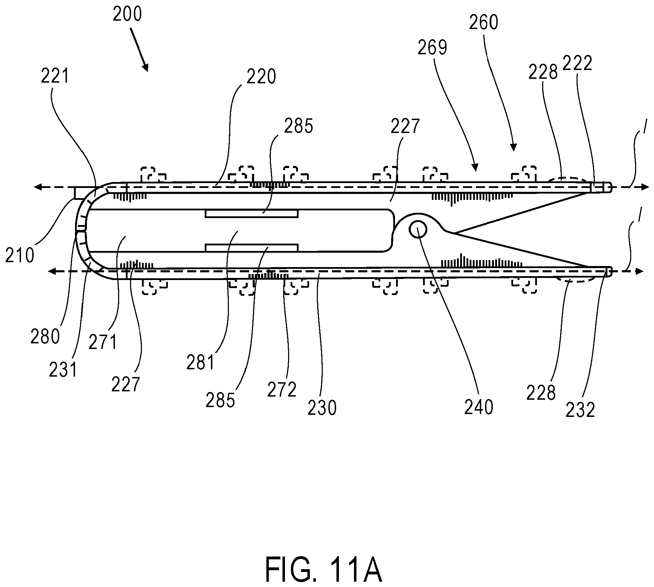

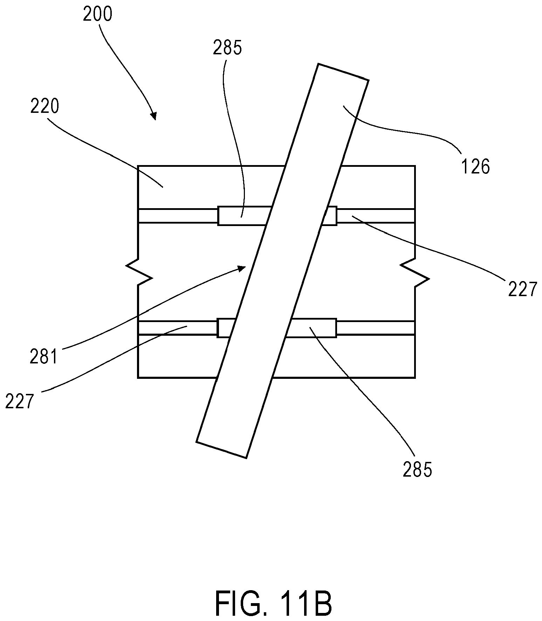

Figures 11A-11B depict an alternative design for the second attachment location 281 in which the second attachment location 281 is static (i.e., not rotatable), but can be attached to differently oriented objects. As depicted in Figures 11A-11B, instead of having structures with rounded indentations 226 that form a cylindrical-shaped attachment location, each arm 220, 230 may include one or more static grips 285 that in combination form the second attachment location 281. The grips 285 are typically positioned about the same distance away from the first ends 221, 231 of their respective arm 220, 230 to thereby align together. Rather than having a rounded shape, each grip typically has a substantially flat shape as depicted in Figure 11A. Each arm 220, 230 may include a pair of flexible grips 285 as depicted in Figure 11B, where each of the grips 285 is attached to or adjacent to one of the ribs 227. Each grip 285 is typically formed of a flexible material, such as rubber. Because of the flat shape of each grip 285, this alternative second attachment location 281 can readily accommodate differently oriented objects, such as the angled pole 126 depicted in Figure 11B. In the closed orientation, the clip 200 typically exerts sufficient force against an object attached at the second attachment location 281 such that the object and clip 200 do not move relative to one another. Thus, changing the orientation of the clip 200 relative to an object typically requires opening the clip 200 to release the object so that the clip 200 and/or object can be moved relative to one another. Thereafter, the clip 200 may be closed to again secure the object in the second attachment location 281.

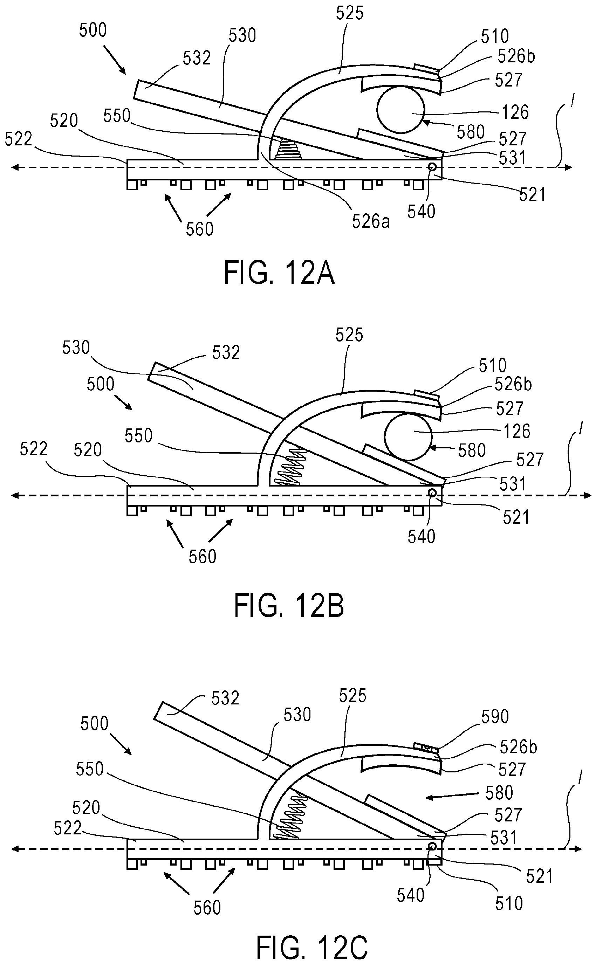

Figures 12A-12C depict a further design for a clip 500 in accordance with the present invention. As depicted in Figure 12A, the clip 500 includes a primary arm 520 and a secondary arm 530. The primary arm 520 typically defines a longitudinal axis *l* between a distal end 521 and a proximal end 522. The primary and secondary arms 520, 530 are typically formed from a rigid material (e.g., a rigid plastic). The primary arm 520 and the secondary arm 530 are pivotally mounted together near (*e.g.,* in proximity to) their respective distal ends 521, 531 at a hinge 540 and their inner sides face together (i.e., face each other) and outer sides face apart (i.e., are opposed and face in substantially opposite directions). The primary arm 520 typically includes a tertiary arm 525 that projects from (or is fixedly attached to) the primary arm (e.g., from the inner side of the primary arm) and typically so that a proximal end 526a of the tertiary arm is connected to the primary arm 520, and the distal end 526b of the tertiary arm 525 extends in the same general direction as the distal end 521 of the primary arm 520 (i.e., away from the proximal end 522 of the primary arm 520), thereby forming a gap between (i) the distal end 526b of the of the tertiary arm 525 and (ii) the distal ends 521, 531 of the primary arm 520 and secondary arm 530 (e.g., between the inner side of the tertiary arm 525 and the inner side of the primary arm 520). As shown in Figures 12A-12C, the tertiary arm 525 may have a curved shape.

The gap between the distal end 531 of the secondary arm 530 and the distal end 526b of the tertiary arm 525 (e.g., between the outer side of the secondary arm 530 and the inner side of the tertiary arm 525) typically forms an attachment location 580. The attachment location 580 is typically sized and configured to attach the clip to a cylindrical-shaped object, such as a pole 126. To facilitate such attachment, the tertiary arm 525 and/or the secondary arm 530 may include a grip 527. Each grip 527 is typically formed from a flexible material. As depicted in Figures 12A-12C, each grip 527 may have a flat shape or a concave shape.

The clip 500 is positionable between a first orientation, typically an open orientation in which there is a larger opening between the outer side of the secondary arm 530 and the inner side of the tertiary arm 525 as depicted in Figure 12A, and a second orientation, typically a closed orientation in which there is a smaller opening between the outer side of the secondary arm 530 and the inner side of the tertiary arm 525 as depicted in Figure 12B. In this regard, the hinge 540 typically facilitates transitioning the primary arms 520 and the secondary arm 530 between the first and second orientations. The proximal ends 522, 532 of the primary arm 520 and the secondary arm 530 are typically smaller distance apart in the open orientation and a larger distance apart in the closed orientation. A biasing member 550 (e.g., a spring) is typically positioned between the primary arm 520 and the secondary arm 530 to bias the clip 500 towards a closed orientation. The biasing member 550 typically applies a biasing force to the primary arm 520 and secondary arm 530 to separate their proximal ends 522, 532 and decrease the size of the opening that forms the attachment location 580 between the secondary arm 530 and the tertiary arm 525. In one embodiment, the biasing member 550 may be a compression spring that (i) applies a force to separate the proximal ends 522, 532 of the primary arm 520 and secondary arm 530 (i.e., resists the proximal ends of the primary and secondary arms being brought closer together) and (ii) compresses when the proximal ends 522, 532 of the primary arm 520 and secondary arm 530 are brought together. As depicted in Figure 12A, to place the clip 500 in the open orientation, a user may exert a force to bring the proximal ends 522, 532 of the primary arm 520 and secondary arm 530 together, thereby increasing the size of the opening between the secondary arm 530 and the tertiary arm 525. Thereafter, an object (e.g., the pole 126) may be placed in the attachment location 580, or the clip 500 may be moved relative to an object already located at the attachment location 580. Once the object is positioned in the attachment location 580, the user may release the proximal ends 522, 532 of the primary arm 520 and secondary arm 530, thereby allowing the biasing member 550 to exert a force separating the proximal ends 522, 532 of the primary arm 520 and secondary arm 530. In addition, the size of the opening between the secondary arm 530 and the tertiary arm 525 is reduced, thereby causing the secondary arm 530 and the tertiary arm 525 to engage the object at the attachment location 580 as depicted in Figure 12B. The force applied by the biasing member 550 is typically sufficient to secure the object within the attachment location 580 and prevent the clip 500 from moving relative to the object until a user compresses when the proximal ends 522, 532 of the primary arm 520 and secondary arm 530 to transition the clip 500 to the open orientation.

One or more connectors 560 are positioned on an outer side of the primary arm 520 (i.e., the side of the primary arm 520 opposite the secondary arm 530) as depicted in Figures 12A-12C. Each connector 560 is configured to receive a transducer (e.g., an arterial blood pressure transducer module). The connectors 560 are typically spaced-apart across the length of outer side of the primary arm 520 to provide adequate spacing to receive each of the transducers. Typically, the connectors 560 are further oriented to direct the incoming and outgoing tubing and cables away from the distal end 521 and proximal end 522 of the primary arm 520 where they could possible by damaged during movement of the clip 500 between the open and closed orientations. Although not depicted in Figures 12A-12C, it is within the scope of the present invention for additional connectors to be positioned along the secondary arm 530 or the tertiary arm 525.

The clip 500 includes an alignment device 510 that is configured to emit an alignment signal that typically causes a light indicia to visually appear on a patient. That said, in some embodiments the alignment signal may not be visible, such as a beam of non-visible electromagnetic radiation (e.g., ultraviolet or infrared radiation). As depicted in Figures 12A-12B and 12C, the alignment device 510 may be attached to the tertiary arm 525 near (*e.g.,* in proximity to) its distal end 526b or to the primary arm 520 near its distal end 521. That said, the alignment device may be attached to other portions of the clip 500. The alignment device 510 may be fixedly attached to the clip 500 (e.g., via an adhesive or one or more screws) or removably attached to the clip 500 (e.g., via a snap fastener). The alignment device 510 is typically attached to the clip 500 so that the alignment device 510 is aligned with the measuring point of any transducers attached to the clip. Some transducers may have differing measuring points. As such, the alignment device may be movable so that it may be aligned with the measuring points associated with different types of transducers. The alignment device 510 may be attached to the clip 500 so that the light beam emitted by the alignment device 510 is substantially parallel to the longitudinal axis *l* of the primary arm 520. In some embodiments, at least a portion of the alignment device 510 may be rotatable relative to the clip 500 in such a way that the light beam can rotate relative to the clip 500. In some particular embodiments, the alignment device 510 may include a self-leveling feature to ensure that the light beam maintains a horizontal alignment regardless of the orientation of the clip 500. As also shown in Figure 12C, the clip 500 includes a bubble level 590 attached to one of the arms that a user can employ to ensure that the clip 500-and in some instances the light beam-is horizontally level. It is also described herein that, rather than the clip 500 including a bubble level, the alignment device 510 may include both (i) a self-leveling feature to ensure that the light beam maintains a horizontal alignment regardless of the orientation of the clip 500 and (ii) a fixed and narrow aperture that only permits passage of the light beam when the light beam is substantially parallel to the longitudinal axis *l* of the of the primary arm 520. By ensuring that (i) both the clip 500 and the light beam are horizontally level and (ii) the light indicia appears at the correct location on a patient (e.g., on the X-mark 132 as depicted in Figure 3), a user can ensure that the measuring points of any attached transducers are vertically aligned with the phlebostatic axis of the patient.

Spatially relative terms such as "under", "below", "lower", "over", "upper", and the like, are used for ease of description to explain the positioning of one element relative to a second element. These terms are intended to encompass different orientations of the device in addition to different orientations than those depicted in the figures. Further, terms such as "first", "second", and the like, are also used to describe various elements, regions, sections, etc and are also not intended to be limiting. Like terms refer to like elements throughout the description.

As used herein, the terms "having", "containing", "including", "comprising" and the like are open ended terms that indicate the presence of stated elements or features, but do not preclude additional elements or features. The articles "a", "an" and "the" are intended to include the plural as well as the singular, unless the context clearly indicates otherwise.

The present disclosure may be carried out in other specific ways than those herein set forth without departing from the scope and essential characteristics of the disclosure. The present embodiments are, therefore, to be considered in all respects as illustrative and not restrictive, and all changes coming within the scope of the appended claims are intended to be embraced therein.

## Claims

1. A medical clip (200; 500) configured to attach to an object, the clip (200; 500) comprising:
a first arm (220; 520) and a second arm (230; 530), each with a first end (221; 231), a second end (222; 232), an inner side, and an outer side, the inner sides of the first (220; 520) and second arms (230; 530) facing each other and the outer sides of the first (220; 520) and second arms (230; 530) being opposed, each of the first arm (220, 520) and the second arm (230; 530) defining a longitudinal axis between its first end (221; 231) and second end (222, 232);
a hinge (240, 540) positioned for transitioning the first (220; 520) and the second arm (230; 530) between a first and a second orientation, the second ends (222; 232) being in closer proximity to each other in the first orientation than in the second orientation;
one or more connectors (260; 560) positioned along the outer side of the first arm (220; 520) and/or the outer side of the second arm (230; 530), each connector (260; 560) being configured to receive at least one medical device; and
an alignment device (210; 510) attached to one of the first or the second arm, the alignment device (210; 510) configured to emit an alignment signal,
wherein the clip (200; 500) includes a bubble level (290; 590) attached to one of the arms (220, 230; 520, 530) that a user can employ to ensure that the clip (200; 500) is horizontally level,
the alignment signal comprises a light beam,
the alignment device (210; 510) comprises a device housing (217) coupled to one of the first (220; 520) and second arms (230; 530) and a light emitting device (216) rotatably coupled to the device housing (217), the light emitting device (216) being configured to emit the light beam; and
when the first (220; 520) and second arm (230; 530) are in a fixed position, the light emitting device (216) is rotatable about an axis orthogonal to a plane that includes the longitudinal axis of the arm (220, 230; 520, 530) coupled to the device housing (217).

2. The clip (200, 500) of claim 1, wherein the alignment device (210;, 510) comprises an aperture (311) that permits the passage of the light beam therethrough when the light beam is substantially parallel to the longitudinal axis of the arm (220, 230; 520, 530) coupled to the device housing (217) but blocks the light beam when the light beam is not substantially parallel to the longitudinal axis of the arm (220, 230; 520, 530) coupled to the device housing (217).

3. The clip (200) of claim 1, further comprising a first structure (227a, 295) located on to the inner side of the first arm (220) and a second structure (227b; 295) located on the inner side of the second arm (230), wherein the first and second structures (227a, 227b; 295) collectively form a first compartment (281) configured to extend substantially around an object in the second orientation.

4. The clip (200) of claim 3, wherein:
the first structure (295) is rotatable relative to the first arm (220), and the second structure (295) is rotatable relative to the second arm (230); and
when the first and the second arms (220, 230) are positioned such that their longitudinal axes are substantially parallel, the first and the second arms (220, 230) are rotatable about an axis (r) substantially orthogonal to two substantially parallel planes that each includes one of the longitudinal axes (I) of the first and the second arms (220, 230).

5. The clip (200) of claim 3, wherein the first compartment (281) forms a substantially cylindrical-shape when the first and second arms (220, 230) are in the second orientation, the substantially cylindrical-shape having an axis (c) substantially parallel with an axis (h) of rotation of the hinge (240).

6. The clip (200) of claim 5, further comprising a third structure (227c) attached to the inner side of the first arm (220) and a fourth structure (227d) attached to the inner side of the second arm (230) and forming a second compartment (282) with a substantially cylindrical-shape having an axis substantially parallel with an axis (h) of rotation of the hinge (240) and a perimeter configured to extend substantially around an object when the first and second arms (220, 230) are in the second orientation, the perimeter of the second compartment (282) being greater than the perimeter of the first compartment (281) when the first and second arms (220, 230) are in the second orientation.

7. The clip (200) of claim 1, further comprising a first structure (295) located on and rotatable relative to the inner side of the first arm (220) and forming at least a portion of a compartment (281) with a perimeter configured to extend around an object in the second orientation;
wherein the first structure (295) is rotatable about an axis (r) substantially orthogonal to a plane that includes the longitudinal axis (I) of the first arm (220).

8. The clip (200) of claim 7, further comprising a second structure (295) located on and rotatable relative to the inner side of the second arm (230) and forming at least a portion of the compartment (281);
wherein the second structure (295) is rotatable about an axis (r) substantially orthogonal to a plane that includes the longitudinal axis (I) of the second arm (230); and
wherein the compartment (281) forms a substantially cylindrical-shape when the first (220) and second arms (230) are in the second orientation.

9. The clip (200; 500) of claim 1, wherein:
the alignment device (210; 510) is fixedly attached to one of the first (220; 520) or the second arms (230; 530) so that the alignment device (210; 510) is configured to emit the light beam along an axis substantially parallel to the longitudinal axis of the arms to which the alignment device (210; 510) is attached.

10. The clip (200) of claim 1, wherein each of the connectors (260) comprises a channel (269) to receive an arterial blood transducer module.

11. The clip (500) of claim 1, wherein
the first arm (520) comprises a third arm (525) extending from the first arm (520) so that (i) a first end (526a) of the third arm (525) is attached to the inner side of the first arm (520), (ii) a second end (521) of the third arm (525) extends in generally the same direction as the second end (521) of the first arm (520), and (iii) an inner side of the third arm (525) and the inner side of the first arm (520) form a space therebetween,
wherein the inner side of the second arm (530) faces the inner side of the first arm (520) and forms a space therebetween,
wherein the outer side of the second arm (530) faces the inner side of the third arm (525) forming a space therebetween that forms an attachment location for an object,
wherein the space between the inner side of the third arm (525) and the outer side of the second arm (530) is greater in the first orientation of the hinge (540) than in the second orientation,
wherein the clip (500) further comprises a biasing member (550) positioned between the first arm (520) and the second arm (530), the biasing member (550) biasing the first and the second arm (530) towards the second orientation,
wherein the one or more connectors (560) is / are positioned along the outer side of the first arm (520), and
wherein the alignment device (510) is attached to one of the first arm (520) or the third arm (525).

## Patentansprüche

1. Medizinische Klemme (200; 500), die zum Anbringen an einem Objekt konfiguriert ist, wobei die Klemme (200; 500) umfasst:
einen ersten Arm (220; 520) und einen zweiten Arm (230; 530), jeweils mit einem ersten Ende (221; 231), einem zweiten Ende (222; 232), einer Innenseite und einer Außenseite, wobei die Innenseiten des ersten Arms (220; 520) und des zweiten Arms (230; 530) einander zugewandt sind und die Außenseiten des ersten Arms (220; 520) und des zweiten Arms (230; 530) gegenüberliegen, wobei jeder von dem ersten Arm (220; 520) und dem zweiten Arm (230; 530) zwischen seinem ersten Ende (221; 231) und seinem zweiten Ende (222; 232) eine Längsachse definiert;
ein Gelenk (240, 540), das für einen Wechsel des ersten Arms (220; 520) und des zweiten Arms (230; 530) zwischen einer ersten und einer zweiten Ausrichtung eingerichtet ist, wobei die zweiten Enden (222; 232) in der ersten Ausrichtung näher beieinander sind als in der zweiten Ausrichtung;
einen oder mehrere Verbinder (260; 560), die entlang der Außenseite des ersten Arms (220; 520) und/oder der Außenseite des zweiten Arms (230; 530) angeordnet sind, wobei jeder Verbinder (260; 560) dafür konfiguriert ist, wenigstens eine medizinische Vorrichtung aufzunehmen; und
eine Ausrichtungsvorrichtung (210; 510), die an einem von dem ersten Arm oder dem zweiten Arm angebracht ist, wobei die Ausrichtungsvorrichtung (210; 510) dafür konfiguriert ist, ein Ausrichtungssignal zu emittieren,
wobei die Klemme (200; 500) eine an einem der Arme (220, 230; 520, 530) angebrachte Wasserwaage (290; 590) aufweist, die ein Benutzer verwenden kann, um sicherzustellen, dass die Klemme (200; 500) horizontal ausgerichtet ist,
wobei das Ausrichtungssignal einen Lichtstrahl umfasst,
wobei die Ausrichtungsvorrichtung (210; 510) ein Vorrichtungsgehäuse (217), das mit einem von dem der ersten Arm (220; 520) und dem zweiten Arm (230; 530) gekoppelt ist, und eine lichtemittierende Vorrichtung (216), die drehbar mit dem Vorrichtungsgehäuse (217) gekoppelt ist, umfasst, wobei die lichtemittierende Vorrichtung (216) dafür konfiguriert ist, den Lichtstrahl zu emittieren; und
wobei dann, wenn der erste Arm (220; 520) und der zweite Arm (230; 530) in einer festen Position sind, die lichtemittierende Vorrichtung (216) um eine Achse drehbar ist, die orthogonal zu einer Ebene ist, die die Längsachse des mit dem Vorrichtungsgehäuse (217) gekoppelten Arms (220, 230; 520, 530) enthält.

2. Klemme (200, 500) nach Anspruch 1, wobei die Ausrichtungsvorrichtung (210; 510) eine Öffnung (311) umfasst, die den Durchtritt des Lichtstrahls ermöglicht, wenn der Lichtstrahl im Wesentlichen parallel zu der Längsachse des mit dem Vorrichtungsgehäuse (217) gekoppelten Arms (220, 230; 520, 530) ist, aber den Lichtstrahl blockiert, wenn der Lichtstrahl nicht im Wesentlichen parallel zu der Längsachse des mit dem Vorrichtungsgehäuse (217) gekoppelten Arms (220, 230; 520, 530) ist.

3. Klemme (200) nach Anspruch 1, die ferner eine erste Struktur (227a, 295), die sich auf der Innenseite des ersten Arms (220) befindet, und eine zweite Struktur (227b; 295), die sich auf der Innenseite des zweiten Arms (230) befindet, umfasst, wobei die erste und die zweite Struktur (227a, 227b; 295) zusammen ein erstes Abteil (281) bilden, das so konfiguriert ist, dass es sich in der zweiten Ausrichtung im Wesentlichen um ein Objekt herum erstreckt.

4. Klemme (200) nach Anspruch 3, wobei:
die erste Struktur (295) relativ zu dem ersten Arm (220) drehbar ist und die zweite Struktur (295) relativ zu dem zweiten Arm (230) drehbar ist; und
dann, wenn der erste und der zweite Arm (220, 230) so positioniert sind, dass ihre Längsachsen im Wesentlichen parallel sind, der erste und der zweite Arm (220, 230) um eine Achse (r) drehbar sind, die im Wesentlichen orthogonal zu zwei im Wesentlichen parallelen Ebenen ist, die jeweils eine der Längsachsen (I) des ersten und des zweiten Arms (220, 230) enthalten.

5. Klemme (200) nach Anspruch 3, wobei das erste Abteil (281) eine im Wesentlichen zylindrische Form bildet, wenn sich der erste und der zweite Arm (220, 230) in der zweiten Ausrichtung befinden, wobei die im Wesentlichen zylindrische Form eine Achse (c) aufweist, die im Wesentlichen parallel zu einer Drehachse (h) des Gelenks (240) verläuft.

6. Klemme (200) nach Anspruch 5, die ferner eine dritte Struktur (227c), die an der Innenseite des ersten Arms (220) angebracht ist, und eine vierte Struktur (227d), die an der Innenseite des zweiten Arms (230) angebracht ist, umfasst und ein zweites Abteil (282) mit einer im Wesentlichen zylindrischen Form bildet, die eine Achse im Wesentlichen parallel zu einer Drehachse (h) des Gelenks (240) und einen Umfang aufweist, der so konfiguriert ist, dass er sich im Wesentlichen um ein Objekt herum erstreckt, wenn sich der erste und der zweite Arm (220, 230) in der zweiten Ausrichtung befinden, wobei der Umfang des zweiten Abteils (282) größer ist als der Umfang des ersten Abteils (281), wenn sich der erste und der zweite Arm (220, 230) in der zweiten Ausrichtung befinden.

7. Klemme (200) nach Anspruch 1, die ferner eine erste Struktur (295) umfasst, die sich an der Innenseite des ersten Arms (220) befindet und relativ zu dieser drehbar ist und wenigstens einen Teil eines Abteils (281) mit einem Umfang bildet, der so konfiguriert ist, dass er sich in der zweiten Ausrichtung um ein Objekt herum erstreckt;
wobei die erste Struktur (295) um eine Achse (r) drehbar ist, die im Wesentlichen orthogonal zu einer Ebene ist, die die Längsachse (I) des ersten Arms (220) enthält.

8. Klemme (200) nach Anspruch 7, die ferner eine zweite Struktur (295) umfasst, die an der Innenseite des zweiten Arms (230) angeordnet und relativ zu dieser drehbar ist und wenigstens einen Teil des Abteils (281) bildet;
wobei die zweite Struktur (295) um eine Achse (r) drehbar ist, die im Wesentlichen orthogonal zu einer Ebene ist, die die Längsachse (I) des zweiten Arms (230) enthält; und
wobei das Abteil (281) eine im Wesentlichen zylindrische Form bildet, wenn sind der erste Arm (220) und der zweite Arm (230) in der zweiten Ausrichtung befinden.

9. Klemme (200; 500) nach Anspruch 1, wobei:
die Ausrichtungsvorrichtung (210; 510) fest an einem von dem ersten Arm (220; 520) oder dem zweiten Arm (230; 530) angebracht ist, so dass die Ausrichtungsvorrichtung (210; 510) dafür konfiguriert ist, den Lichtstrahl entlang einer Achse zu emittieren, die im Wesentlichen parallel zu der Längsachse der Arme ist, an denen die Ausrichtungsvorrichtung (210; 510) angebracht ist.

10. Klemme (200) nach Anspruch 1, wobei jeder der Verbinder (260) eine Nut (269) zur Aufnahme eines arteriellen Blut-Wandlermoduls umfasst.

11. Klemme (500) nach Anspruch 1, wobei
der erste Arm (520) einen dritten Arm (525) umfasst, der sich ausgehend von dem ersten Arm (520) erstreckt, so dass (i) ein erstes Ende (526a) des dritten Arms (525) an der Innenseite des ersten Arms (520) angebracht ist, (ii) ein zweites Ende (521) des dritten Arms (525) sich im Allgemeinen in der gleichen Richtung erstreckt wie das zweite Ende (521) des ersten Arms (520), und (iii) eine Innenseite des dritten Arms (525) und die Innenseite des ersten Arms (520) dazwischen einen Raum bilden,
wobei die Innenseite des zweiten Arms (530) der Innenseite des ersten Arms (520) zugewandt ist und dazwischen einen Zwischenraum bildet,
wobei die Außenseite des zweiten Arms (530) der Innenseite des dritten Arms (525) zugewandt ist und dazwischen einen Zwischenraum bildet, der eine Befestigungsstelle für ein Objekt bildet,
wobei der Raum zwischen der Innenseite des dritten Arms (525) und der Außenseite des zweiten Arms (530) in der ersten Ausrichtung des Gelenks (540) größer ist als in der zweiten Ausrichtung,
wobei die Klemme (500) ferner ein Vorspannelement (550) umfasst, das zwischen dem ersten Arm (520) und dem zweiten Arm (530) angeordnet ist, wobei das Vorspannelement (550) den ersten und den zweiten Arm (530) in Richtung zu der zweiten Ausrichtung vorspannt,
wobei der eine oder die mehreren Verbinder (560) entlang der Außenseite des ersten Arms (520) angeordnet ist/sind, und
wobei die Ausrichtungsvorrichtung (510) an einem von dem ersten Arm (520) oder dem dritten Arm (525) angebracht ist.

## Revendications

1. Clip médical (200 ; 500) configuré pour fixer à un objet, le clip (200 ; 500) comprenant :
un premier bras (220 ; 520) et un deuxième bras (230 ; 530), chacun avec une première extrémité (221 ; 231), une deuxième extrémité (222 ; 232), une face intérieure et une face extérieure, la face intérieure des premier (220 ; 520) et deuxième bras (230 ; 530) se faisant face et la face extérieure des premier (220 ; 520) et deuxième bras (230 ; 530) étant opposées, chacun des premier (220, 520) et deuxième bras (230 ; 530) définissant un axe longitudinal entre leur première extrémité (221 ; 231) et deuxième extrémité (222 ; 232) ;
une charnière (240, 540) positionnée pour le passage des premier (220 ; 520) et deuxième bras (230 ; 530) entre une première et une deuxième orientations, les deuxièmes extrémités (222 ; 232) étant plus près l'une de l'autre dans la première orientation que dans la deuxième orientation ;
un ou plusieurs connecteurs (260 ; 560) positionnés le long de la face extérieure du premier bras (220 ; 520) et/ou de la face extérieure du deuxième bras (230 ; 530), chaque connecteur (260 ; 560) étant configuré pour recevoir au moins un dispositif médical ; et
un dispositif d'alignement (210 ; 510) fixé à un du premier ou du deuxième bras, le dispositif d'alignement (210 ; 510) étant configuré pour émettre un signal d'alignement,
le clip (200 ; 500) comportant un niveau à bulle (290 ; 590) fixé à un des bras (220, 230 ; 520, 530) qu'un utilisateur peut employer pour assurer que le clip (200 ; 500) est à l'horizontale,
le signal d'alignement comprenant un faisceau lumineux,
le dispositif d'alignement (210 ; 510) comprenant un boîtier de dispositif (217) couplé à un du premier (220 ; 520) et deuxième bras (230 ; 530) et un dispositif électroluminescent (216) couplé de manière rotative au boîtier de dispositif (217), le dispositif électroluminescent (216) étant configuré pour émettre le faisceau lumineux ; et
lorsque les premier (220 ; 520) et deuxième bras (230 ; 530) sont en position fixe, le dispositif électroluminescent (216) peut tourner autour d'un axe orthogonal à un plan qui comporte l'axe longitudinal du bras (220, 230 ; 520, 530) couplé au boîtier de dispositif (217).

2. Clip (200, 500) selon la revendication 1, dans lequel le dispositif d'alignement (210 ; 510) comprend une ouverture (311) qui permet le passage du faisceau lumineux à travers elle lorsque le faisceau lumineux est essentiellement parallèle à l'axe longitudinal du bras (220, 230 ; 520, 530) couplé au boîtier de dispositif (217), mais bloque le faisceau lumineux lorsque le faisceau lumineux n'est pas essentiellement parallèle à l'axe longitudinal du bras (220, 230 ; 520, 530) couplé au boîtier de dispositif (217).

3. Clip (200) selon la revendication 1, comprenant en outre une première structure (227a, 295) située sur la face intérieure du premier bras (220) et une deuxième structure (227b ; 295) située sur la face intérieure du deuxième bras (230), dans lequel les première et deuxième structures (227a, 227b ; 295) forment ensemble un premier compartiment (281) configuré pour s'étendre essentiellement autour d'un objet dans la deuxième orientation.

4. Clip (200) selon la revendication 3, dans lequel :
la première structure (295) peut tourner par rapport au premier bras (220) et la deuxième structure (295) peut tourner par rapport au deuxième bras (230) ; et
lorsque les premier et deuxième bras (220, 230) sont positionnés de telle sorte que leur axes longitudinaux sont essentiellement parallèles, les premier et deuxième bras (220, 230) pouvant tourner autour d'un axe (r) essentiellement orthogonal aux deux plans essentiellement parallèles qui chacun comporte un des axes longitudinaux (I) des premier et deuxième bras (220, 230).

5. Clip (200) selon la revendication 3, dans lequel le premier compartiment (281) est formé essentiellement comme un cylindre lorsque les premier et deuxième bras (220, 230) sont dans la deuxième orientation, la forme essentiellement cylindrique ayant un axe (c) essentiellement parallèle à un axe (h) de rotation de la charnière (240).

6. Clip (200) selon la revendication 5, comprenant en outre une troisième structure (227c) fixée à la face intérieure du premier bras (220) et une quatrième structure (227d) fixée à la face intérieure du deuxième bras (230) et formant un deuxième compartiment (282) avec une forme essentiellement cylindrique ayant un axe essentiellement parallèle à un axe (h) de rotation de la charnière (240) et un périmètre configuré pour s'étendre essentiellement autour d'un objet lorsque les premier et deuxième bras (220, 230) sont dans la deuxième orientation, le périmètre du deuxième compartiment (282) étant supérieur au périmètre du premier compartiment (281) lorsque les premier et deuxième bras (220, 230) sont dans la deuxième orientation.

7. Clip (200) selon la revendication 1, comprenant en outre une première structure (295) située sur et pouvant tourner par rapport à la face intérieure du premier bras (220) et formant au moins une partie d'un compartiment (281) avec un périmètre configuré pour s'étendre autour d'un objet dans la deuxième orientation ;
dans lequel la première structure (295) peut tourner autour d'un axe (r) essentiellement orthogonal à un plan qui comporte l'axe longitudinal (I) du premier bras (220).

8. Clip (200) selon la revendication 7, comprenant en outre une deuxième structure (295) située sur et pouvant tourner par rapport à la face intérieure du deuxième bras (230) et formant au moins une partie du compartiment (281) ;
dans lequel la deuxième structure (295) peut tourner autour d'un axe (r) essentiellement orthogonal à un plan qui comporte l'axe longitudinal (I) du deuxième bras (230) ; et
dans lequel le compartiment (281) est formé essentiellement comme un cylindre lorsque les premier (220) et deuxième bras (230) sont dans la deuxième orientation.

9. Clip (200 ; 500) selon la revendication 1, dans lequel :
le dispositif d'alignement (210 ; 510) est solidarisé à un du premier (220 ; 520) ou du deuxième bras (230 ; 530) de telle sorte que le dispositif d'alignement (210 ; 510) est configuré pour émettre le faisceau lumineux le long d'un axe essentiellement parallèle à l'axe longitudinal des bras auxquels le dispositif d'alignement (210 ; 510) est fixé.

10. Clip (200) selon la revendication 1, dans lequel chacun des connecteurs (260) comprend un canal (269) afin de recevoir un module transducteur de sang artériel.

11. Clip (500) selon la revendication 1, dans lequel
le premier bras (520) comprend un troisième bras (525) s'étendant du premier bras (520) de telle sorte qu' (i) une première extrémité (526a) du troisième bras (525) est fixée à la face intérieure du premier bras (520), (ii) une deuxième extrémité (521) du troisième bras (525) s'étend généralement dans la même direction que la deuxième extrémité (521) du premier bras (520) et (iii) une face intérieure du troisième bras (525) et la face intérieure du premier bras (520) forment un espace entre elles,
dans lequel la face intérieure du deuxième bras (530) fait face à la face intérieure du premier bras (520) et forme un espace entre elles,
dans lequel la face extérieure du deuxième bras (530) fait face à la face intérieure du troisième bras (525) formant un espace entre elles qui forme un point de fixation pour un objet,
dans lequel l'espace entre la face intérieure du troisième bras (525) et la face extérieure du deuxième bras (530) est supérieur dans la première orientation de la charnière (540) que dans la deuxième orientation,
dans lequel le clip (500) comprend en outre un élément de sollicitation (550) positionné entre le premier bras (520) et le deuxième bras (530), l'élément de sollicitation (550) sollicitant le premier et le deuxième bras (530) vers la deuxième orientation,
dans lequel l'un ou les plusieurs connecteurs (560) est/sont positionné(s) le long de la face extérieure du premier bras (520) et
dans lequel le dispositif d'alignement (510) est fixé à un du premier bras (520) ou du troisième bras (525).
